# EUROPÄISCHE PATENTSCHRIFT

(11) **EP 0 553 651 B1**
(45) Veröffentlichungstag und Bekanntmachung des Hinweises auf die Patenterteilung: **31.01.1996**
(21) Anmeldenummer: 93100482.4
(22) Anmeldetag: 14.01.1993
(51) Int. Cl.: C07C 311/17, C07C 335/08, C08K 5/43, C08L 75/06, C08G 65/32, C09J 171/02

(54) **Sulfonamide und deren Verwendung**
Sulfonamides and their use
Sulfonamides et leur utilisation

(30) Priorität: 14.01.1992 DE 4200768
(43) Veröffentlichungstag der Anmeldung: 04.08.1993
(73) Patentinhaber: DR. TH. BÖHME KG CHEM. FABRIK GMBH & CO., D-82538 Geretsried (DE)
(72) Erfinder: Balbach, Günther, Dr., W-8190 Wolfratshausen (DE); Döcker, Horst, W-8192 Geretsried (DE)
(74) Vertreter: Fehners, Klaus Friedrich, Dipl.-Ing., Dipl.-Wirtsch.-Ing.

(56) Entgegenhaltungen:
- EP-A- 0 265 753
- DE-B- 2 049 802
- US-A- 2 577 256
- US-A- 2 740 814
- CHEMISCHE BERICHTE Bd. 109, 1976, Seiten 1803 - 1831 E. WEBER
- E.W. FLICK 'PLASTIC ADDITIVES, An Industrial Guide' 1986 , NOYES PUBLICATIONS , PARK RIDGE, NEW JERSEY, US

## Beschreibung

Die Erfindung betrifft neuartige Sulfonamide und deren Verwendung für die Modifizierung von Kunststoffen.

Sulfonamide werden für viele Anwendungen industriell hergestellt. Neben der wohlbekannten Verwendung in der Pharmazie spielt die technische Verwendung als Kunststoffweichmacher eine bedeutende Rolle.

Kunststoff-Weichmacher im engeren Sinne sind Lösungsmittel, welche so hochsiedend sind, daß sie bei der Verarbeitung und beim Gebrauch im Kunststoff verbleiben und durch ihre Anwesenheit einen permanenten Quellungszustand erzeugen. Dadurch wird die Kohäsion der den Kunststoff aufbauenden Moleküle verringert, was sich makroskopisch in einer größeren Verformbarkeit bei Einwirkung derselben Kraft und in visko-elastischem Verhalten zeigt. Der Kunststoff erscheint weicher.

Eine weitere Folge der verminderten Kohäsion ist ein niedrigerer Schmelzpunkt und eine verminderte Schmelzviskosität. Diese Veränderungen sind beim Formulieren von Schmelzklebern von entscheidender Bedeutung.

Für eine gute Solvatisierung der Kunststoff-Moleküle muß die Polarität des Weichmachers dem jeweiligen Kunststoff angepaßt sein. Für Polyvinylacetat, Polyurethane, Polyamide und thermoplastische Polyester haben sich flüssige Sulfonamide als gut geeignet erwiesen. Allerdings haben flüssige Sulfonamide, z.B. das technisch übliche Gemisch von N-Ethyl-p-toluolsulfonamid und N-Ethyl-o-toluolsulfonamid, den Nachteil, daß trotz des hohen Siedepunktes dieser Flüssigkeiten bei der Verarbeitungstemperatur der Kunststoffe teilweise Verdampfung eintritt. Werden mit derartigen Sulfonamiden weichgemachte Kunststoffe im Automobilbau für Teile des Innenraums verwendet, kommt es durch die Verdampfung zum sogenannten "window-fogging", einem Niederschlag von Weichmachernebeln auf den Fensterflächen. Auch in anderen Industriebereichen ist die Bildung derartiger Nebel aus Gründen des Arbeits- und Umweltschutzes unerwünscht.

Versucht man, den Dampfdruck, und dadurch die Flüchtigkeit dieser Stoffe, durch Erhöhung des Molekulargewichtes zu vermindern, erhält man durch die hohe Kristallisationstendenz der Sulfonamide feste Produkte. Diese müssen für die Verwendung als Kunststoff-Weichmacher besonders aufbereitet werden (mahlen und ggfs. sieben), um eine gleichmäßige Vermischung zu ermöglichen. Eine Weichmach-Wirkung ist bei kristallisierenden Substanzen nur dann zu erwarten, wenn die Solvatisierung des Kunststoffes energetisch günstiger ist als die Kristallisation.

Dieser Erfindung liegt die Aufgabe zugrunde, flüssige Weichmacher auf Sulfonamidbasis mit niedrigem Dampfdruck zu finden, welche mit Polyvinylacetat, Polyurethanen, Polyamiden und thermoplastischen Polyestern verträglich sind.

Es hat sich überraschenderweise gezeigt, daß die Sulfonamide der allgemeinen Formel 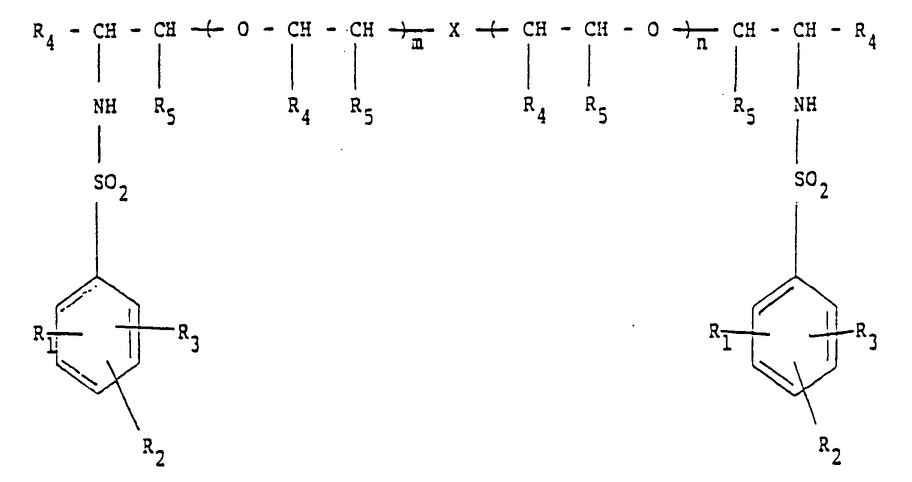
worin
R₁, R₂ und R₃ jeweils unabhängig voneinander Wasserstoff, fakultativ substituiertes C₁-C₄-Alkyl, fakultativ substituiertes C₁-C₄-Alkoxy, fakultativ substituiertes Phenoxy oder fakultativ substituiertes C₁-C₄-Acyloxy sein können,
R₄ und R₅ entweder beide Wasserstoff bedeuten oder eine von Zwei vicinal angeordneten Gruppen R₄ und R₅ Wasserstoff und die andere C₁-C₄-Alkyl darstellen,
-X- Sauerstoff, die Gruppe -NH-C(O)-NH- oder die Gruppe -NH-C(S)-NH bedeutet und m und n jeweils eine ganze Zahl von 1 bis 40 sind (diese Sulfonamide
mit Ausnahme von N,N'-Ditoluolsulfonyl -3,6,9 -trioxa - 1,11 - undecandiamin sind neu), hochsiedende Flüssigkeiten sind, welche mit polaren Polymeren wie Polyvinylacetat, Polyamid, thermoplastischen Polyestern und Polyurethanen verträglich sind, diese Kunststoffe weichmachen und den Schmelzpunkt und die Schmelzviskosität herabsetzen. Die Flüchtigkeit der neuen Disulfonamide ist um Größenordnungen geringer als die der üblichen flüssigen Monosulfonamide.

Die Disulfonamide lassen sich erfindungsgemäß durch Umsetzen der entsprechenden α,Ω-Diaminopolyethylenglykole bzw. N,N'-(Ω-Aminopolyethylenglykol)-harnstoffe oder - thioharnstoffe in wäßriger, basischer Lösung mit den entsprechenden Benzolsulfonsäurechloriden herstellen.

Folgende Beispiele sollen die Erfindung besser erläutern, ohne sie auf das spezielle Beispiel zu beschränken.

### Beispiel 1

### Herstellung eines erfindungsgemäßen Disulfonamides

In 700 g Wasser werden 127,2 g calcinierte Soda auf gelöst. Diese Lösung wird in einem Dreihalskolben mit Rüherer und Tropftrichter mit 460 g eines α-Ω-Aminopolypropylenglykols des mittleren Molekulargewichtes 400 vermischt, und in diese Mischung wird unter Rühren 353,2 g Benzol-Sulfochlorid eingetropft. Dabei wird darauf geachtet, daß 45°C in der Reaktionsmischung nicht überschritten werden. Wenn alles Sulfochlorid zugesetzt ist, wird noch 2 Std. nachgerührt, wobei durch Heizen eine Temperatur zwischen 35°C und 45°C aufrechterhalten wird.

Die Mischung wird in einen Scheidetrichter überführt. Es bilden sich 2 Schichten. Die Unterschicht ist das Sulfonamid. Es wird abgetrennt, mit 700 g einer 1% Ammoniaklösung gewaschen und wieder abgetrennt.

Wasserreste im Disulfonamid werden durch Vakuumdestillation entfernt. Das Disulfonamid ist eine hellgelbe hochviskose Flüssigkeit.

### Beispiel 2

### Flüchtigkeit der Disulfonamide

Ein wesentlicher technischer Vorteil bei Verwendung der erfindungsgemäßen Disulfonamide ist die geringe Flüchtigkeit. Dies soll am Produkt des Beispiels 1 im Vergleich mit einem handelsüblichen flüssigen Monosulfonamid aufgezeigt werden. (N-Ethyl-o,p-Toluolsulfonamid mit 75 % Orthoanteil).

**Tabelle für Beispiel 2:**

| **GEWICHTSVERLUST** BEI 150°C | | |
|---|---|---|
| | 15 Minuten | 30 Minuten |
| N-Ethyl-o,p-Toluolsulfonamid | 1,6 % | 3,2 % |
| Erfindungsgemäßer Weichmacher nach Beispiel 1 | 0,2 % | 0,4 % |

Zur Bestimmung der in obiger Tabelle angegebenen Werte wurden mit der Analysenwaage 15 g des zu untersuchenden Weichmachers in eine Petrischale von 9 cm Durchmesser eingewogen. Nach der in der Tabelle angegebenen Wärmebehandlung wurde auf Zimmertemperatur abgekühlt und zurückgewogen.

### Beispiel 3

Die plastifizierende Wirkung der neuen Disulfonamide wurde durch Einarbeitung in einen aus wäßriger Dispersion aufgebrachten Schmelzkleber und Beurteilung der Klebefestigkeit bei verschiedenen Temperaturen geprüft. Eine Plastifizierung des im Kleber verwendeten Polymers zeigt sich in einer verbesserten Haftung bei niedrigen Verklebungstemperaturen. Auch hier wurde ein Vergleich mit der technisch bewährten Mischung N-Ethyl-o,p-Toluolsulfonamid durchgeführt.

### a- Herstellung eines geeigneten Dispergiermittels

75,9 g Wasser werden gerührt und 2 g einer handelsüblichen löslichen Stärke eingestreut. Es wird gerührt, bis die Stärke sich glatt gelöst hat. Diese Lösung wird auf 60°C erhitzt. Parallel dazu werden 10 g eines Reaktionsproduktes von 1 mol Stearylalkohol mit 4 mol Ethylenoxyd aufgeschmolzen. Die Schmelze wird der Stärkelösung zugesetzt und unter kräftigem Rühren abgekühlt. Es entsteht eine stabile weiße Dispersion. Jetzt werden 3 g Polyvinylpyrrolidon von einem mittleren Molekulargewicht Mᵥ = 38 000 Dalton gelöst in 9 g Wasser unter Rühren zugesetzt. Da die Masse sehr schaumig ist, werden 2 g eines handelsüblichen Silikonentschäumers zugegeben.

### b- Herstellung der Schmelzkleberpasten

| Rezeptur Nr. | 1 | 2 | 3 |
|---|---|---|---|
| Dispergiermittel nach 3a- | 24 | 24 | 24 |
| Wasser | 41 | 41 | 41 |
| N-Ethyl-o, p-Toluol-Sulfonamid | - | 7 | - |
| Weichmacher aus Beispiel 1 | - | - | 7 |
| 5 %ige Lösung eines handelsüblichen Diurethanverdickers *) | 3 | 3 | 3 |
| 25 %ige Lösung des Na-Salzes eines handelsüblichen Polyacrylsäureverdickers **) | 3 | 3 | 3 |
| handelsübliches Pulver eines thermoplast. Polyesters +) | 35 | 35 | 35 |
| 1 % ige Lösung eines hochmolekularen Polkyethylenglykols ++) | 10 | 10 | 10 |

| | | | |
|---|---|---|---|
| *) = ATESYNTH® 5112 (Dr. Th. Böhme KG) | | | |
| **) = LATECOLL® (BASF), neutralisiert | | | |
| +) = GRILTEX® 8 P 1 (EMS-CHEMIE) | | | |
| ++) = MIRAPLAST® 5147 (Dr. Th. Böhme KG) | | | |

### c- Aufbringen des Klebers auf ein Substrat

Mit einer 17 mesh Metallschablone wurden die Pasten 1 - 3 auf ein Polyamidvlies punktförmig aufgetragen und das bedruckte Vlies 1 min. bei 120°C in einem Heizofen mit Umluft getrocknet.

### d- Klebekraftprüfung

Die beschichteten Vliese wurden auf einer Durchlaufpresse mit einem Oberstoff aus 72 % Viskose und 28 % Polyestermikrofasern verpreßt. Die Temperatur in der Klebefuge betrug 120°C und 110°C, die Durchlaufzeit durch die Presse 10,5 sec und der Walzendruck 4 bar.

Die Kraft für das Abziehen eines 5 cm breiten Streifens des Oberstoffes von der Vlieseinlage wurde gemessen, und zwar
- ohne weitere Behandlung
- nach einer Feinwäsche bei 40°C
- und nach einer chemischen Reinigung mit Perchlorethylen.

Folgende Haftwerte wurden erhalten:

| **VERKLEBUNG BEI 120°C** | | | |
|---|---|---|---|
| Rezeptur Nr. | 1 | 2 | 3 |
| Primärhaftung | 3 N | 5 N | 5 N |
| Nach Feinwäsche | 0,8 N | 5 N | 5 N |
| Nach chem. Reinigung | 3 N | 6 N | 6 N |

| **VERKLEBUNG BEI 110°C** | | | |
|---|---|---|---|
| Rezeptur Nr. | 1 | 2 | 3 |
| Primärhaftung | 2 N | 4 N | 5 N |
| Nach Feinwäsche | 0 N | 4 N | 6 N |
| Nach chem. Reinigung | 1,5 N | 4 N | 6 N |

Die angegebenen Haftwerte sind Mittelwerte aus 5 Messungen über jeweils 15 cm Länge. Aus den Haftwerten ist die plastifizierende Wirkung des erfindungsgemäßen Disulfonamids klar zu erkennen. Sie ist mit der Wirkung des wesentlich leichter flüchtigen flüssigen Monosulfonamids vergleichbar.

### Beispiel 4

### Weichmachende Wirkung in Polyurethan

Zum Nachweis der Weichmacher-Wirkung wurden verschiedene Mengen der nach Beispiel 1 hergestellten Substanz in Polyurethanfilme eingearbeitet. Zur Herstellung der Filme wurden zwei verschiedene 25%ige Lösungen von thermoplastischen Polyurethanen auf Polyesterbasis hergestellt. Diese Lösungen wurden mit den in beiliegenden Tabellen genannten Mengen an Weichmachern versetzt. Die Prozentangaben beziehen sich auf das gelöste Polyurethan.

Bei einem Filmstreifen von 0,04 mm Dicke und 15 mm Breite wurde die notwendige Zugkraft für die Erreichung einer bestimmten Dehnung des Films und die Reißdehnung bestimmt.

Die Ergebnisse sind in den Tabellen 1 und 2 dargestellt. Die weichmachende Wirkung zeigt sich in einer Abnahme der Zugkraft. Die gleichzeitig beobachtbare Erhöhung der Reißdehnung zeigt, daß die Filme nicht zu viel von ihrer Kohäsion verloren haben.

**Tabelle 1 für Beispiel 4:**

| **Thermoplastisches Einkomponenten-Polyurethan** ***)** | |
|---|---|
| **Weichmacherkonzentration** | **Zugkraft/ N /** |
| **37,5 % Dehnung** | |
| 0 % | 3,0 |
| 9 % | 2,6 % |
| 23 % | 2,4 % |

| **75 % Dehnung** | |
|---|---|
| 0 % | 3,6 % |
| 9 % | 3,4 % |
| 23 % | 2,9 % |

| **Reißdehnung** | |
|---|---|
| **Weichmacherkonzentration** | **Reißdehnung (%)** |
| 0 % | 428 |
| 4 % | 475 |
| 23 % | 575 |

| | |
|---|---|
| *) Impranil EHC® (Bayer) | |

**Tabelle 2 für Beispiel 4:**

| **Polyurethan auf Polyesterbasis** ***)** | |
|---|---|
| **Weichmacherkonzentration** | **Zugkraft/ N /** |
| **37,5 % Dehnung** | |
| 0 % | 5,4 % |
| 9 % | 3,8 % |
| 23 % | 2,8 % |
| 33 % | 2,5 % |

| **75 % Dehnung** | |
|---|---|
| 0 % | 6,4 % |
| 9 % | 4,5 % |
| 23 % | 3,4 % |
| 33 % | 3,1 % |

| **Reißdehnung** | |
|---|---|
| **Weichmacherkonzentration** | **Reißdehnung %** |
| 0 % | 388 |
| 9 % | 417 |
| 23 % | 463 |
| 33 % | 538 |

| | |
|---|---|
| *) Estane 5707® (Tiefenbacher & Co., Hamburg) | |

## Patentansprüche

1. Verwendung einer oder mehrerer Verbindung(en) der allgemeinen Formel: 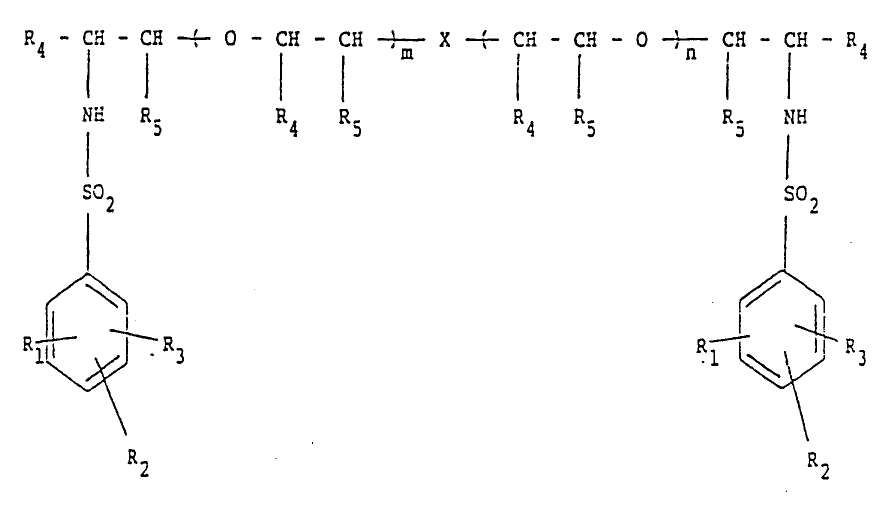 worin
R₁, R₂ und R₃ jeweils unabhängig voneinander Wasserstoff, fakultativ substituiertes C₁-C₄-Alkyl, fakultativ substituiertes C₁-C₄-Alkoxy, fakultativ substituiertes Phenoxy oder fakultativ substituiertes C₁-C₄-Acyloxy sein können,
R₄ und R₅ entweder beide Wasserstoff bedeuten oder eine von zwei vicinal angeordneten Gruppen R₄ und R₅ Wasserstoff und die andere C₁-C₄-Alkyl darstellen,
-X- Sauerstoff, die Gruppe -NH-C(O)-NH oder die Gruppe -NH-C(S)-NH bedeutet und m und n jeweils eine ganze Zahl von 1 bis 40 sind,
für die Modifzierung von Kunststoffen.

2. Verwendung nach Anspruch 1, **dadurch gekennzeichnet**, daß in den Verbindungen R₁ Wasserstoff oder C₁-C₄-Alkyl bedeutet und R₂ und R₃ jeweils Wasserstoff sind, und/oder daß in den Verbindungen -X- Sauerstoff ist.

3. Verwendung nach Anspruch 2, **dadurch gekennzeichnet**, daß in den Verbindungen R₁, R₂ und R₃ jeweils Wasserstoff bedeuten.

4. Verwendung einer oder mehrerer Verbindung(en) nach einem der Ansprache 1 bis 3 als Weichmacher für polare Polymere.

5. Verwendung einer oder mehrerer der Verbindungen nach einem der Ansprüche 1 bis 3 zur Erniedrigung des Schmelzpunktes und der Schmelzviskosität von polaren Polymeren.

6. Verwendung nach Anspruch 4 oder 5, **dadurch gekennzeichnet**, daß die Polymeren ausgewählt sind unter Homo- und Copolymeren, die Polyamide, thermoplastische Polyester, Polyvinylacetate oder Polyurethane oder Mischungen davon enthalten oder aus diesen bestehen.

7. Verbindungen der allgemeinen Formel,
worin 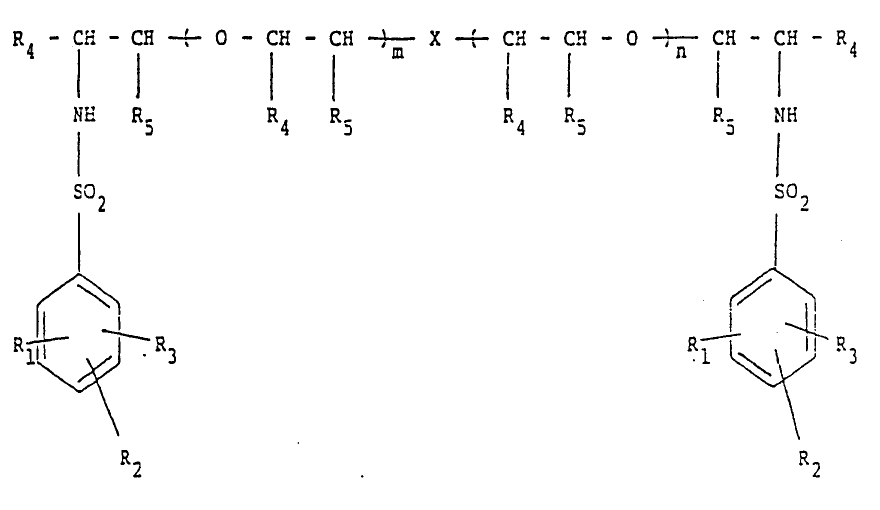 R₁, R₂ und R₃ jeweils unabhängig voneinander Wasserstoff, fakultativ substituiertes C₁-C₄-Alkyl, fakultativ substituiertes C₁-C₄-Alkoxy, fakultativ substituiertes Phenoxy oder fakultativ substituiertes C₁-C₄-Acyloxy sein können,
R₄ und R₅ entweder beide Wasserstoff bedeuten oder eine von zwei vicinal angeordneten Gruppen R₄ und R₅ Wasserstoff und die andere C₁-C₄-Alkyl darstellen,
-X- Sauerstoff, die Gruppe -NH- C(O)-NH oder die Gruppe -NH-C(S)-NH bedeutet und m und n jeweils eine ganze Zahl von 1 bis 40 sind,
mit Ausnahme von N,N'-Ditoluolsulfonyl -3,6,9- trioxa -1,11- undecandiamin.

8. Verbindungen nach Anspruch 7, **dadurch gekennzeichnet**, daß R₁ Wasserstoff oder C₁-C₄-Alkyl bedeutet und R₂ und R₃ jeweils Wasserstoff sind, und/oder daß -X- Sauerstoff ist.

9. Verfahren zum Herstellen von Verbindungen nach einem der Ansprüche 7 oder 8, worin -X-Sauerstoff ist, **dadurch gekennzeichnet**, daß ein fakultativ an den Ethylengruppen C₁-C₄-alkylsubstituiertes alpha-omega-Diaminopolyethylenglycol in einer wäßrigen, basischen Lösung mit einem Benzolsulfonsäurechlorid vermischt wird, welches mit einem bis drei Substituenten, ausgewählt unter fakultativ substituiertem C₁-C₄-Alkyl, fakultativ substituiertem C₁-C₄-Alkoxy, fakultativ substituiertem Phenoxy oder fakultativ substituiertem C₁-C₄-Acyloxy, substituiert sein kann, wobei die Reaktionsmischung gegebenenfalls gekühlt wird, und nach ausreichender Nachreaktionszeit das Sulfonamid abgetrennt, gegebenenfalls gewaschen und getrocknet wird.

10. Verfahren zum Herstellen von Verbindungen nach einem der Ansprüche 7 oder 8, worin -X-NH-C(O)-NH- bzw. -NH-C(S)-NH- ist, dadurch gekennzeichnet, daß ein fakultativ an den Ethylengruppen C₁-C₄-alkylsubstituierter N,N'-Di(omega-aminopolyethylen-glycol) harnstoff oder -thioharnstoff in einer wäßrigen, basischen Lösung mit einem Benzolsulfonsäurechlorid vermischt wird, welches mit einem bis drei Substituenten, ausgewählt unter fakultativ substituiertem C₁-C₄-Alkyl, fakultativ substituiertem C₁-C₄-Alkoxy, fakultativ substituiertem Phenoxy oder fakultativ substituiertem C₁-C₄-Acyloxy, substituiert sein kann, wobei die Reaktionsmischung gegebenenfalls gekühlt wird, und nach ausreichender Nachreaktionszeit das Sulfonamid abgetrennt, gegebenenfalls gewaschen und getrocknet wird.

## Claims

1. Use of one or more compounds having the general formula: 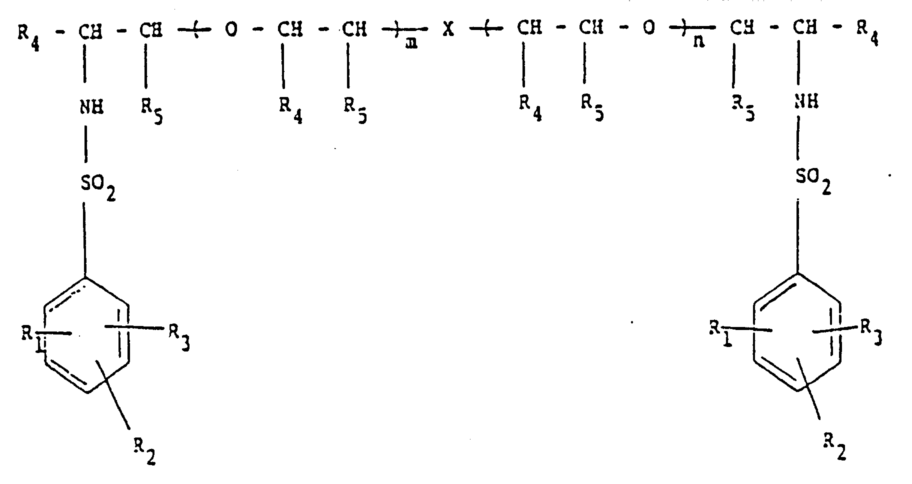 wherein
R₁, R₂ and R₃ can independently denote hydrogen, optionally substituted C₁-C₄ alkyl, optionally substituted C₁-C₄ alkoxy, optionally substituted phenoxy or optionally substituted C₁-C₄ acyloxy,
R₄ and R₅ either both represent hydrogen or one of two neighbouring groups R₄ and R₅ denotes hydrogen and the other denotes C₁-C₄ alkyl,
-X- denotes oxygen, the group -NH-C(O)-NH or the group -NH-C(S)-NH and m and n each denote an integer from 1 to 40,
for the modification of plastics.

2. Use according to claim 1, characterised in that in the compounds R₁ denotes hydrogen or C₁-C₄ alkyl and R₂ and R₃ each denote hydrogen and/or -X- in the compounds denotes oxygen.

3. Use according to claim 2, characterised in that R₁, R₂ and R₃ in the compounds each denote hydrogen.

4. Use of one or more compounds according to any of claims 1 to 3 as plasticisers for polar polymers.

5. Use of one or more of the compounds according to any of claims 1 to 3 for lowering the melting point and the melt viscosity of polar polymers.

6. Use according to claim 4 or 5, characterised in that the polymers are selected from among homopolymers and copolymers which contain or consist of polyamides, thermoplastic polyesters, polyvinyl acetates, polyurethanes or mixtures thereof.

7. Compounds having the general formula: 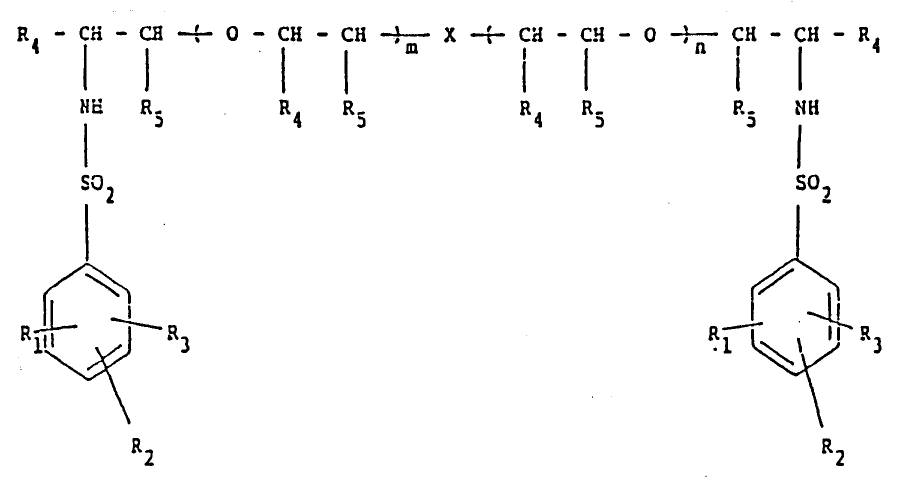 wherein
R₁, R₂ and R₃ can independently denote hydrogen, optionally substituted C₁-C₄ alkyl, optionally substituted C₁-C₄ alkoxy, optionally substituted phenoxy or optionally substituted C₁-C₄ acyloxy,
R₄ and R₅ either both represent hydrogen or one of two neighbouring groups R₄ and R₅ denotes hydrogen and the other denotes C₁-C₄ alkyl,
-X- denotes oxygen, the group -NH-C(O)-NH or the group -NH-C(S)-NH and m and n each denote an integer from 1 to 40,
with the exception of N,N'-ditoluene sulphonyl-3,6,9-trioxa-1,11-undecane diamine.

8. Compounds according to claim 7, characterised in that R₁ denotes hydrogen or C₁-C₄ alkyl and R₂ and R₃ each denote hydrogen and/or -X- is oxygen.

9. A process for the preparation of compounds according to claim 7 or 8, wherein -X- denotes oxygen, characterised in that an alpha-omega diaminopolyethylene glycol optionally C₁-C₄-alkyl-substituted at the ethylene groups is mixed in an aqueous basic solution with a benzene sulphonic acid chloride which can be substituted with 1 to 3 substituents selected from among optionally substituted C₁-C₄ alkyl, optionally substituted C₁-C₄ alkoxy, optionally substituted phenoxy or optionally substituted C₁-C₄ acyloxy, the reaction mixture being cooled if required, and after a sufficient time after the reaction, the sulphonamide is separated, washed if required and dried.

10. A process for the preparation of compounds according to claim 7 or 8, wherein -X- denotes NH-C(O)-NH- or -NH-C(S)-NH-, characterised in that an N,N'-di(omega-aminopolyethylene glycol) urea or thiourea optionally C₁-C₄-alkyl substituted at the ethylene groups is mixed in an aqueous basic solution with a benzene sulphonic acid chloride which can be substituted by 1 to 3 substituents selected from among optionally substituted C₁-C₄-alkyl, optionally substituted C₁-C₄-alkoxy, optionally substituted phenoxy or optionally substituted C₁-C₄ acyloxy, the reaction mixture being cooled if required and, after a sufficient time after the reaction, the sulphonamide is separated, washed if required and dried.

## Revendications

1. Utilisation d'un ou plusieurs composé(s) de formule générale : 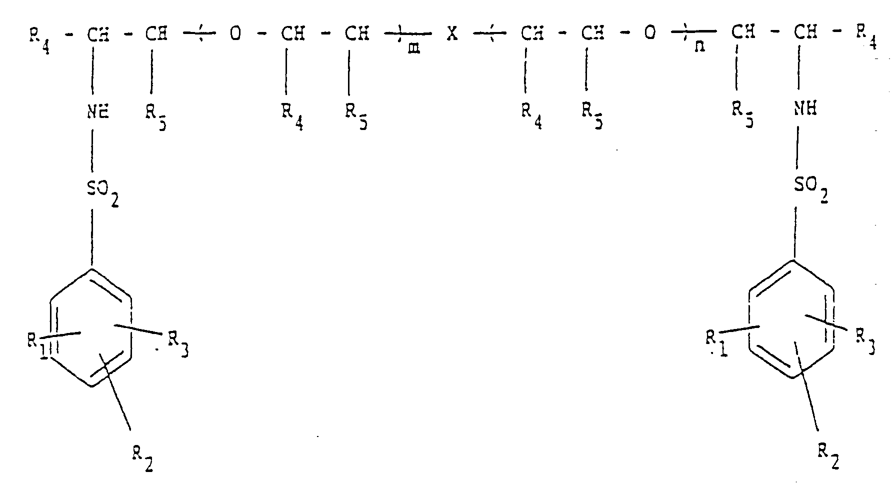 dans laquelle
R₁, R₂ et R₃ peuvent être chacun, indépendamment les uns des autres, de l'hydrogène, un alkyle en C₁ à C₄ à substitution facultative, un alkoxy en C₁ à C₄ à substitution facultative, un phénoxy à substitution facultative ou un acyloxy en C₁ à C₄ à substitution facultative,
R₄ ou R₅ sont soit les deux de l'hydrogène soit l'un des deux groupes R₄ et R₅ disposés de façon vicinale de l'hydrogène et l'autre groupe un alkyle en C₁ à C₄,
la liaison -X- est l'oxygène, le groupe -NH-C(O)-NH ou le groupe -NH-C(S)-NH, m et n étant chacun un nombre entier compris entre 1 et 40,
pour la modification de matières synthétiques.

2. Utilisation selon la revendication 1, caractérisée en ce que, dans les composés, R₁ est l'hydrogène ou un alkyle en C₁ à C₄ et R₂ et R₃ sont chacun de l'hydrogène, et/ou en ce que dans les composés, -X- est l'oxygène.

3. Utilisation selon la revendication 2, caractérisée en ce que dans les composés R₁, R₂ et R₃, sont chaque fois de l'hydrogène.

4. Utilisation d'un ou plusieurs composés selon l'une des revendications 1 à 3 à titre de plastifiants pour des polymères polaires.

5. Utilisation d'un ou plusieurs des composés selon l'une des revendications 1 à 3, pour abaisser le point de fusion et la viscosité en fusion de polymères polaires.

6. Utilisation sel on la revendication 4 ou 5, caractérisée en ce que les polymères sont choisis dans les homo et copolymères, les polyamides, les polyesters thermoplastiques, les polyvinylacétates ou les polyuréthannes ou contiennent leurs mélanges ou bien en sont constitués.

7. Composés de formule générale, 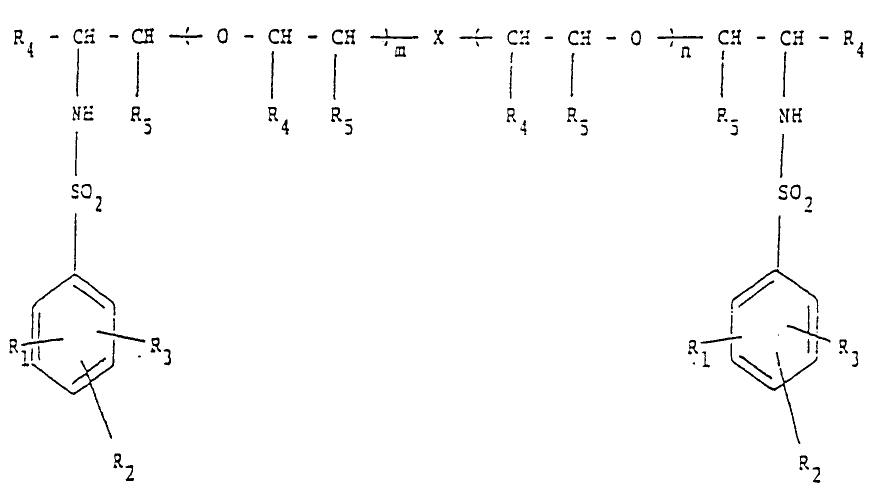 dans laquelle
R₁, R₂ et R₃ sont chaque fois indépendamment les uns des autres de l'hydrogène, un alkyle en C₁ à C₄ à substitution facultative, un alkoxy en C₁ à C₄ à substitution facultative, un phénoxy à substitution facultative ou un acyloxy en C₁ à C₄ à substitution facultative,
R₄ et R₅ sont soit les deux de l'hydrogène soit l'un des deux groupes R₄ et R₅ disposés de façon vicinale de l'hydrogène et l'autre groupe un alkyle en C₁ à C₄,
-X- est l'oxygène, le groupe -NH-C(O)-NH ou le groupe - NH-C(S)-NH, m et n étant chacun un nombre entier compris entre 1 et 40,
à l'exception de la N,N'-ditoluolsulfonyle-3,6,9-trioxa-1,11-undécandiamine.

8. Composés selon la revendication 7, caractérisés en ce que R₁ est l'hydrogène ou un alkyle en C₁ à C₄ et R₂ et R₃ sont chacun l'hydrogène et/ou en ce que -X- est de l'oxygène.

9. Procédé de préparation de composés sel on l'une des revendications 7 ou 8, dans lequel l'élément -X- est de l'oxygène, caractérisé en ce qu'on mélange un alpha-oméga-diaminopolyéthylène glycol à substitution facultative du groupe alkyle sur les groupes éthylène C₁ à C₄ dans une solution aqueuse basique avec un chlorure d'acide benzosulfonique pouvant être substitué par un à trois substituants, choisi parmi l'alkyle en C₁ à C₄ à substitution facultative, l'alkoxy en C₁ à C₄ à substitution facultative, le phénoxy à substitution facultative ou l'acyloxy en C₁ à C₄ à substitution facultative, le mélange de réaction étant, le cas échéant, refroidi puis, une fois le temps de postréaction écoulé, le sulfonamide étant séparé, le cas échéant lavé et séché.

10. Procédé de préparation de composés selon l'une des revendications 7 ou 8, dans lequel l'élément -X-est NH-C(O)-NH ou NH-C(S)-NH, caractérisé en ce qu'une N,N'-(omega-aminopolyéthylène-glycol) urée ou thio-urée substituée en alkyle C₁ à C₄ sur les groupes éthylène est mélangée dans une solution aqueuse basique avec un chlorure d'acide benzosulfonique qui peut être substitué par un à trois composants, choisis parmi l'alkyle en C₁ à C₄ à substitution facultative, l'alkoxy en C₁ à C₄ à substitution facultative, le phénoxy à substitution facultative ou l'acyloxy en C₁ à C₄ à substitution facultative, le mélange de réaction étant le cas échéant refroidi et, après écoulement d'un temps de postréaction suffisant, le sulfonamide est séparé, le cas échéant lavé et séché.
